# EUROPEAN PATENT SPECIFICATION

(11) **EP 1 868 581 B1**
(45) Date of publication and mention of the grant of the patent: **14.03.2012**
(21) Application number: 06740898.9
(22) Date of filing: 11.04.2006
(51) Int. Cl.: A61K 9/20, A61K 31/47, A61P 31/04, A61K 9/48

(54) **PHARMACEUTICAL COMPOSITIONS HAVING IMPROVED DISSOLUTION PROFILES FOR POORLY SOLUBLE DRUGS**
PHARMAZEUTISCHE ZUSAMMENSETZUNGEN MIT VERBESSERTEN AUFLÖSEPROFILEN FÜR SCHWERLÖSLICHE WIRKSTOFFE
COMPOSITIONS PHARMACEUTIQUES PRESENTANT DES PROFILS DE DISSOLUTION AMELIORES POUR DES MEDICAMENTS PEU SOLUBLES

(30) Priority: 11.04.2005 US 670207 P
(43) Date of publication of application: 26.12.2007
(60) Divisional application: 10178627.5
(73) Proprietor: Abbott Laboratories, Abbott Park, Illinois 60064-6008 (US)
(72) Inventor: WU, Huailiang, Long Grove, IL 60047 (US); ZHANG, Geoff, Libertyville, IL 60048 (US)
(74) Representative: Modiano, Micaela Nadia
(86) International application number: PCT/US2006/013651
(87) International publication number: WO 2006/110815

(56) References cited:
- EP-A- 0 257 320
- EP-A- 0 411 619
- EP-A- 0 852 140
- EP-A- 1 219 618
- DE-A1- 2 213 275
- US-A- 4 748 174
- US-A- 6 133 284
- PATENT ABSTRACTS OF JAPAN vol. 2003, no. 12, 5 December 2003 (2003-12-05) & JP 2003 300882 A (ST MARIANNA UNIV SCHOOL OF MEDICINE; MUKKU:KK; DAI ICHI SEIYAKU CO LTD), 21 October 2003 (2003-10-21)

## Description

### FIELD OF THE INVENTION

This invention pertains to pharmaceutical compositions having improved dissolution profiles for drugs therein.

### BACKGROUND OF THE INVENTION

Absorption of poorly soluble drugs is a major obstacle in pharmaceutical development. For ionizable compound salt formation is often used to improve solubility. This approach, however, may not work for compounds that precipitatate at lower pH, such as gastric pH. There is therefore an existing need in the pharmaceutical development arts for compositions which provide improved dissolution profiles of drugs.

### BRIEF DESCRIPTION OF THE FIGURES

FIG. 1 shows dissolution profiles of formulations containing various amounts of crystallization inhibitor for EXAMPLE 1.
FIG. 2 shows dissolution profiles of formulations containing a pH modifier and a crystallization inhibitor for EXAMPLE 1.
FIG. 3 shows dissolution profiles of formulations containing apt modifier and a crystallization inhibitor for 5,5-diphenylhydantoin, sodium salt.
FIG. 4 shows dissolution profiles of formulations containing a pH modifier and a crystallization inhibitor for potassium mefenamate.

### SUMMARY OF THE INVENTION

The present invention provides a composition comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof, a crystallization inhibitor selected from the group consisting of hydroxypropylmethylcellulose, polyvinylpyrrolidone, and hydroxypropylcellulose, and a pH modifier, said composition providing a measurable improvement in dissolution rate of the 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof.

The present invention also provides the use of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof, a crystallization inhibitor selected from the group consisting of hydroxypropylmethylcellulose, polyvinylpyrrolidone, and hydroxypropylcellulose, and a pH modifier for the manufacture of a composition providing a measurable improvement in dissolution rate of the 1-(6-amino-3,5-difluoropyridin-2-yl)8-chloro-6-fluoro-7-(3-hydroxyazetidin-1-yl)-4-oxo-1,4-dihydroquinoline-3-carboxylic acid or a salt thereof for treating disease in a patient by administering thereto said composition.

In one embodiment of the above composition or use, the salt of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid comprises meglumine.

### DETAILED DESCRIPTION OF THE INVENTION

The term "crystallization inhibitor," as used herein, means an agent that facilitates prevention of precipitation of a drug in a composition comprising the agent, a pH modifier and the drug. The crystallization inhibitors used in the present invention are hydroxypropylmethylcellulose, polyvinypyrrolidone, hydroxypropylcellulose.

The term "ionizable drug," as used herein, means a compound having an acidic or basic moiety that is useful for treating a disease state or an adverse physiological event.

The term "measurable improvement in dissolution rate," as used herein, means at least a 5% increase in dissolution rate at a particular temperature and pH.

The term "pH modifier," as used herein, means a compound that is capable of changing the pH of a solution. Examples of pH modifiers include, but are not limited to NaOH, LiOH, KOH, Na₂CO₃, NaHCO₃, K₂CO₃, KHCO₃, NaH₂PO4, Na₂HPO₄, Na₃PO₄, KH₂PO₄, K₂HPO₄, K₃PO₄, megluamine, Ca(OH)₂, Mg(OH)₂, Zn(OH)₂, Al(OH)₃, pyridoxine, triethanolamine, ammonium hydroxide, cytosine, diethylamine, meglumine, ornithine, glycine, lysine, arginine, valine, proline, aspartic acid, alaninc, asparagine, isoleucine, leucine, methionine, threonine, choline hydroxide, procaine, diethylethanolamine, glucasamine, guanine, nicotinamide, piperazine, guanidinc, olamine, piperidine, triethylamine, tromethaminc, bcnzathinc, benzathine, adenine, mixtures thereof and the like.

The term "pharmaceutical compositions," as used herein, means a combination of substances, at least one of which is a drug, or a therapeutically acceptable salt thereof.

The term "q.s.," as used herein, means as much as suffices.

1-(6-amino-3,5-difluoropyridin-2-yl)-8-chloro-6-fluoro-7-(3-hydroxyazetidin-1-yl)-4-oxo-1,4-dihydroquinoline-3-carboxylic acid may exist as acid addition salts, basic addition salts or zwitterions. Salts of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid are prepared during their isolation or following their purification. Acid addition salts are those derived from the reaction of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid with acid. Accordingly, salts including the acetate, adipate, alginate, bicarbonate, citrate, aspartate, benzoate, benzenesulfonate, bisulfate, butyrate, camphorate, camphorsulfonate, digluconate, formate, fumarate, glycerophosphate, glutamate, hemisulfate, heptanoate, hexanoate, hydrochloride, hydrobromide, hydroiodide, lactobionate, lactate, maleate, mesitylenesulfonate, methanesulfanate, megluamine, naphthylenesulfonate, nicotinate, oxalate, pamoate, pectinate, persulfatc, phosphate, picrate, propionate, succinate, tartrate, thiocyanate, trichloroacetic, trifluoroacetic, paratoluenesulfonate and undecanoate salts of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid are meant to be embraced by this invention. Basic addition salts of compounds are those derived from the reaction of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid with the bicarbonate, carbonate, hydroxide or phosphate of cations such as lithium, sodium, potassium, calcium and magnesium.

1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof may be administered, for example, bucally, ophthalmically, orally, osmotically, parenterally (intramuscularly, intraperintoneally intrasternally, intravenously, subcutaneously), rectally, topically, transdermally, vaginally and intraarterially as well as by intraarticular injection, infusion, and placement in the body, such as, for example, the vasculature.

Therapeutically effective amounts of 1-(6-amino-3,5-difluoropyridin-2-yl)-8-chloro-6-fluoro-7-(3-hydroxyazetidin-1-yl)-4-oxo-1,4-dihydroquinoline-3-caxboxylic acid or a salt thereof depend on recipient of treatment, disease treated and severity thereof, composition comprising it, time of administration, route of administration, duration of treatment, potency, rate of clearance and whether or not another drug is co-administered. The amount of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof used to make a composition to be administered daily to a patient in a single dose or in divided doses is from about 0.03 to about 200 mg/kg body weight. Single dose compositions contain these amounts or a combination of submultiples thereof.

1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof may be administered with or without an excipient. Excipients include, but are not limited to, encapsulators and additives such as absorption accelerators, antioxidants, binders, buffers, coating agents, coloring agents, diluents, disintegrating agents, emulsifiers, extenders, fillers, flavoring agents, humectants, lubricants, perfumes, preservatives, propellants, releasing agents, sterilizing agents, sweeteners, solubilizers, wetting agents, mixtures thereof and the like.

Excipients for preparation of compositions comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof to be administered orally include, but are not limited to, agar, alginic acid, aluminum hydroxide, benzyl alcohol, benzyl benzoate, 1,3-butylene glycol, carbomers, castor oil, cellulose, cellulose acetate, cocoa butter, corn starch, corn oil, cottonseed oil, crosspovidone, diglycerides, ethanol, ethyl cellulose, ethyl laureate, ethyl oleate, fatty acid esters, gelatin, germ oil, glucose, glycerol, groundnut oil, isopropanol, isotonic saline, lactose, magnesium hydroxide, magnesium stearate, malt, mannitol, monoglycerides, olive oil, peanut oil, potassium phosphate salts, potato starch, povidone, propylene glycol, Ringers solution, safflower oil, sesame oil, sodium carboxymethyl cellulose, sodium phosphate salts, sodium lauryl sulfate, sodium sorbitol, soybean oil, stearic acids, stearyl fumarate, sucrose, surfactants, talc, tragacanth, tetrahydrofurfuryl alcohol, triglycerides, water, mixtures thereof and the like. Excipients for preparation of compositions comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof to be administered ophthalmically or orally include, but are not limited to, 1,3-butylene glycol, castor oil, corn oil, cottonseed oil, ethanol, fatty acid esters of sorbitan, germ oil, groundnut oil, glycerol, isopropanol, olive oil, polyethylene glycols, propylene glycol, sesame oil, water, mixtures thereof and the like, Excipients for preparation of compositions comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof to be administered osmotically include, but are not limited to, chlorofluorohydrocarbons, ethanol, water, mixtures thereof and the like. Excipients for preparation of compositions comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof to be administered parenterally include, but are not limited to, 1,3-butanediol, - castor oil, corn oil, cottonseed oil, dextrose, germ oil, groundnut oil, liposomes, oleic acid, olive oil, peanut oil, Ringer's solution, safflower oil, sesame oil, soybean oil, U.S.P or isotonic sodium chloride solution, water, mixtures thereof and the like. Excipients for preparation of compositions comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof to be administered rectally or vaginally include, but are not limited to, cocoa butter, polyethylene glycol, wax, mixtures thereof and the like.

### EXAMPLE 1

### 1-(6-amino-3,5-difluoropyridin-2-yl)-8-chloro-6-fluoro-7-(3-hydroxyazetidin-1-yl)-4-oxo-1,4-dihydroqninoline-3-carboxylic acid

This compound was prepared as described in US 6,133,284,

### EXAMPLE 2

### 1-deoxy-1-(methylammonium)-D-glucitol 1-(6-amino-3,5-difluoropyridin-2-yl)-8-chloro-6-fluoro-7-(3-hydroxyazetidin-1-yl)-4-oxo-1,4-dihydroquinoline-3-carbaxylate

A mixture of EXAMPLE 1 (29.6Kg) and 1-deoxy-1-(methylamino)-D-glucitol (18.4Kg) was/diluted with water (133Kg), stirred at 60°C until all solids dissolved, cooled to 38°C and held there until solid formed, cooled to 0°C and filtered. The filtrant was washed with isopropanol and dried at 50°C.

Ingredients were weighed out according to the formulas described in the tables of components and compositions of control tablet or capsule formulations shown in TABLE 1 TABLE 2 and TABLE 3. Polyvinylpyrrolidine (PVP) or hydroxypropylmethylcelluose (HPMC) in 15.5 mL (25% of total batch size: 50 g) of water. Mixtures of EXAMPLE 1 and Na₂CO₃ were dry mixed in a granulator for 5 minutes, treated with PVP or HPMC solution over 1 minute with water addition to assure optimal granulation, dried at 50°C until the loss on drying (LOD) was less than 1% (typically18 hours) and passed through a 20 mesh screen. The screened powder was blended with the extragranular for 3 minutes. The tablets were compressed to a hardness of 18-20 SCU using a Carver presser with tooling #A2201 Record compression forces or manually filled into appropriate size capsules to provide a fill of 500 mg/unit.

For the single pH dissolution medium experiment, 0.1 N HCl (pH 1.0) or 0.1 N HCl (900 mL) with various concentrations of HPMC (0.001%-1%) at 37°C ± 0.5°C were used with a USP Dissolution Apparatus 2 at a speed of 50 or 100 ± 4% rpm. One test tablet or capsule was added to the medium, and 10 mL of samples were removed at 15, 30, 45, 60, 120 and 240 minutes and assayed by UV absorption at 282 nm.

For the dual pH dissolution medium experiment, one test tablet or capsule was added to 0.1 N HCl (500 mL) at 37°C ± 0.5°C. Dissolution samples were removed at 15, 30, 45, 60, 120 and 240 minutes and assayed by UV absorption at 282 nm, and the dissolution medium was treated with 0.11 8M sodium phosphate buffer (pre-warmed to 37°C ± 0.5°C).

**TABLE 1**

| Ingredient | Formulation A | Formulation B | Formulation C | Formulation D |
|---|---|---|---|---|
| Intergranular | | | | |
| EXAMPLE 1 | 288.6 | 288.6 | 288.6 | 288.6 |
| HPMC | | | | 144.3 (powder) |
| PVP | 15.0 (powder) | 15.0 (soln) | 15.0 (soln) | |
| Na₂CO₃ | | | 75.0 | |
| Crospovidone | 25.0 | | | |
| Avicel | 144.0 | | | |
| water | q.s. | q.s. | q.s. | |

| Extragranular | | | | |
|---|---|---|---|---|
| Avicel | | 93.4 | 93.4 | |
| Crospovidone | 25.0 | 25.0 | 25.0 | |
| Mg Stearate | 2.5 | 2.5 | 2.5 | |
| | | | | |
| Tablet Wt. (mg) | 500 | 425 | 425 | 433.3 |

**TABLE 2**

| Ingredient | Formulation E (mg) | Formulation F (mg) | Formulation G (mg) | Formulation H (mg) |
|---|---|---|---|---|
| Intergranular | | | | |
| EXAMPLE 1 | 288.6 | 288.6 | 288.6 | 288.6 |
| HPMC E5 | 15.0 (soln.) | | 15.0 (soln.) | 15.0 (soln.) |
| PVP k30 | | 15.0 (soln.) | | |
| Na₂CO₃ | 75 | 75 | 75 | 75 |

| Extragranular | | | | |
|---|---|---|---|---|
| HPMCE5 | | 10 | 10 | 25 |
| Avicel PH 102 | 93.9 | 83.9 | 83.9 | 68.9 |
| Crospovidone | 25 | 25 | 5 | 25 |
| Mg Stearate | 2.5 | 2.5 | 2.5 | 2.5 |
| | | | | |
| Tablet Wt. (mg) | 500 | 500 | 500 | 500 |
| Hardness (SCU) | 18-20 | 18-20 | 18-20 | 18-20 |

**TABLE 3**

| Ingredient (mg) | Formulations | | |
|---|---|---|---|
| | Control | Polymer Solution | Polymer Powder |
| Intergranular | | | |
| ABT-492(salt)* | 288.6 | 288.6 | 288.6 |
| Polymer | | 0.5-100 | 2.5-15 |
| water | q.s. | q.s. | q.s. |

| Extragranular | | | |
|---|---|---|---|
| Avicel | 183.9 | 83.9-183.4 | 168.9-181.4 |
| Crospovidone | 25 | 25 | 25 |
| Mg Stearate | 2.5 | 2.5 | 2.5 |
| Tablet wt. (mg) | 500 | 500 | 500 |

| | | | |
|---|---|---|---|
| *megluamine | | | |

Little or no drug was released in the low pH (1.2) medium within one hour for all tested formulations. Following adjustment to pH 7.5 for 2 hours, release of the drug from the control tablets were about 20% and almost the same as that from the formulation containing 0.1% polyvinyl pyrrolidinone (PVP K30). Dissolution of the drug was improved with an increase in amount ofPVP K30. High dissolution extent (>80%) was obtained for formulations containing 1-3% PVP K30. Inclusion of hydroxypropylmethylcelluose (HPMC E5) showed a similar impact on drug dissolution.

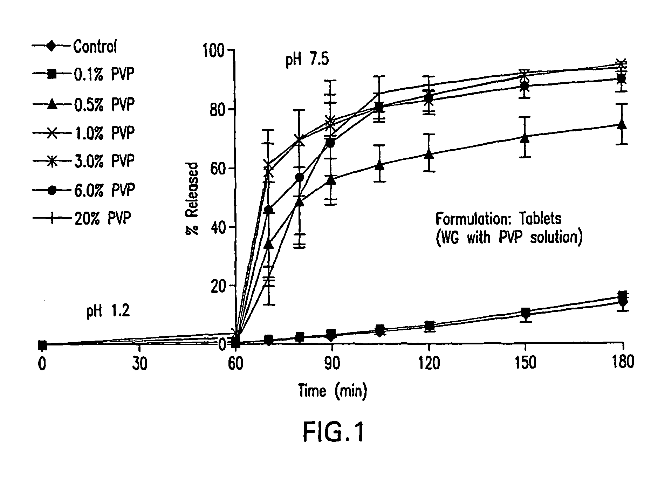

For the tablets made by adding PVP solution as granulation liquid with no Na₂CO₃, dissolution increases with moderate variability in spite of the release pH 1.0 medium being low. Average percentage released increases to 85% in higher pH medium at the 2 hour time point. Direct compression of drug with a higher percentage of HPMC (E5 LV) resulted in a reduction in dissolution variability although a large amount of HPMC would be expected to slow dissolution rate. These results are summarized in Figure 1.

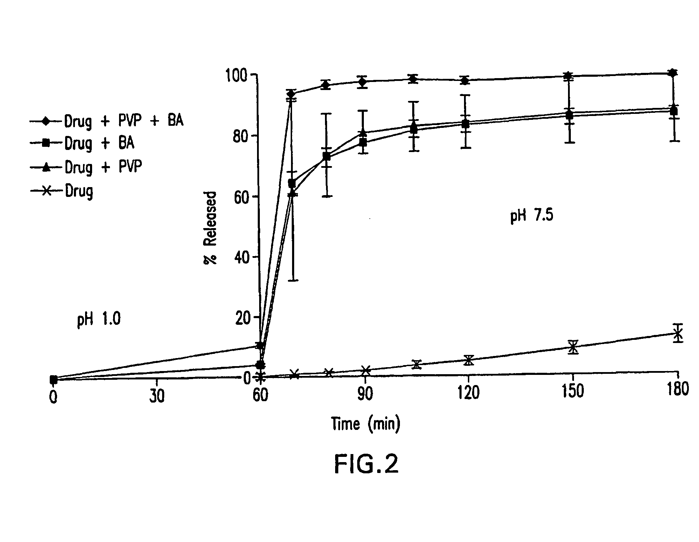

Dissolution of tablets granulated with PVP solution and Na₂CO₃ is rapid in the dual pH media. More interestingly, in pH 1.0 medium, there was about 12% of drug released at the 1 hour time point with an EXAMPLE 1 concentration about 20-fold greater than its intrinsic solubility. The pH of the solution at this time point was 1.0. The differences in dissolution rate/extant and variability among the formulations tested are summarized in TABLE 3 with data from the last pull point at pH 7.5 used as the example. These results are summarized in Figure 2.

**TABLE 4**

| | No Na₂CO₃ PVP Powder | No Na₂CO₃ PVP Solution | Na₂CO₃ PVP Solution |
|---|---|---|---|
| Percent EXAMPLE 1 Released at 2 hours | 78.28% | 88.37% | 99.77% |
| RSD* at 2 hours (percentage) | 55.7% | 12.2% | 0.51% |

| | | | |
|---|---|---|---|
| *relative standard deviation | | | |

These data show the increased dissolution of a drug in the presence of a pH modifier and polymer. As seen in Figure 2, there is little drug released into the low pH medium even after increasing the duration of the capsules in pH 1.0. However, duration time in low pH media does effect the dissolution profile of EXAMPLE 1 in the dual pH media after the pH has been adjusted to 7.5. In general, the shorter exposure time in low pH resulted in a higher extent dissolution.

Similar experiments as a comparative example were conducted with 5,5-diphenylhydantoin, sodium salt (NaPh). The solubility of 5,5-diphenylhydantoin at varying pH's is reported in J. Pharm. Sci. Vol. 82(3), March 1993. The dissolution study was conducted in pH 7 buffer (900 mL) at 37°C ± 0.5°C with a paddle rotation speed of 50 rpm. Samples were removed at pre-determined time points and analyzed for concentration or drug. Formulations of NaPh are shown in TABLE 5.

**TABLE 5**

| Formulation: | B | F | G | H | I | J |
|---|---|---|---|---|---|---|
| Ingredients | | | | | | |
| NaPh | 3 g | 3 g | 3 g | 3 g | 3 g | 3 g |
| NaOH | - | 420 mg | 420 mg | 420 mg | - | - |
| PVP | - | - | 1.2 g | 1.2 g | - | - |
| Lactose | - | - | | 3 g | 3 g | 3 g |
| Water (adj. to pH 7 with NaOH) q.s. | 3L | 3L | 3L | 3L | 3L | 3L |

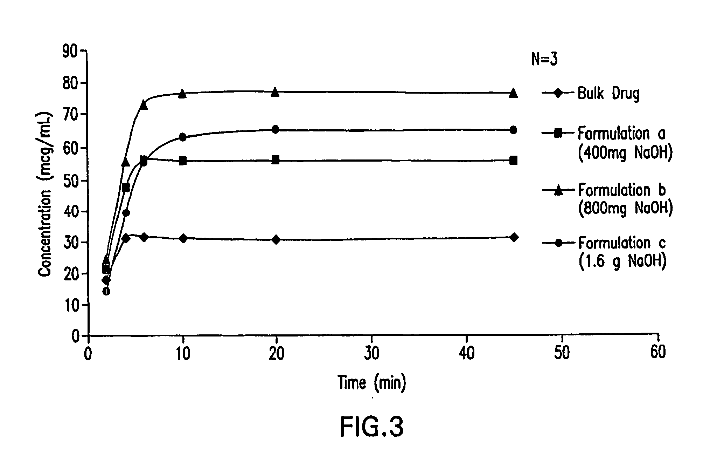

The results of the study, shown in Figure 3, illustrate the higher extent of dissolution. These data stand in contrast to the observation that 5,5-diphenylhydantoin, sodium salt forms an insoluble plug during dissolution at lower pH.

**TABLE 6**

| Ingredient. (mg) | MEF | MEF-K | MEF-K+ BA | MEF-K+ Polymer | MEF-K+ BA+PVP | MEF-K+ BA+ HPMC |
|---|---|---|---|---|---|---|
| MEF | 100 | - | - | - | - | - |
| MEF-K | - | 115 | 117.3 | 115 | 117.3 | 117.3 |
| Na₂CO₃ (BA) | - | - | 28.2-48.9 | - | 29.2-48.9 | 29.2-48.9 . |
| Polymer (in solution) | - | - | 4.8 | 4.8 | 3.1-12.2 | 6.1 |
| Tablet weight (mg) | 190 | 115 | 150-180 | 120 | 153-183 | 153-183 |

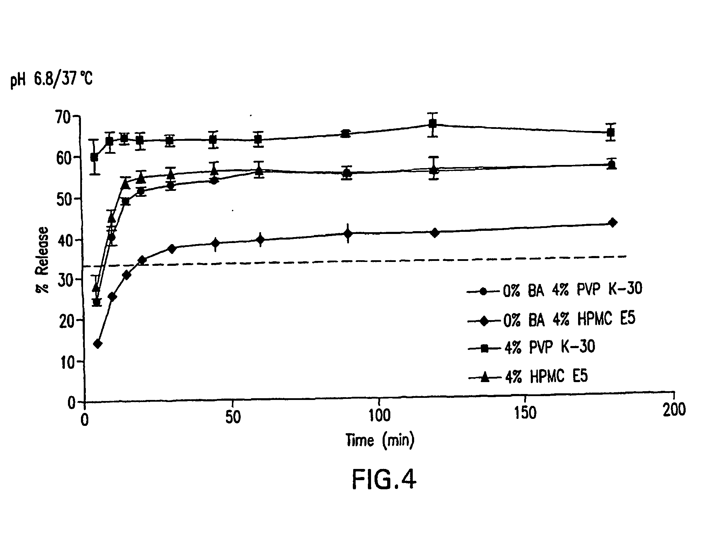

Similar experiments as a further comparative example were conducted with potassium mefenamate (MEF-K). The intrinsic solubility (aqueous medium/lower pH) of mefanamic, acid is 0.0423 µg/mL. The dissolution study was conducted in pH 6.8 buffer (900 mL) at 37°C ± 0.5°C with a paddle rotation speed of 50 rpm. Samples were removed at pre-determined time points and analyzed for concentration or drug. The results of this experiment are shown in Figure 4 and illustrate the improved dissolution of the formulation comprising potassium mefenamate. In this case, the concentration in the dissolution medium exceeded the solubility of the drug.

## Claims

1. A composition comprising 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof, a crystallization inhibitor selected from the group consisting of hydroxypropylmethylcellulose, polyvinylpyrrolidone, and hydroxypropylcellulose, and a pH modifier, said composition providing a measurable improvement in dissolution rate of the 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof

2. Use of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof, a crystallization inhibitor selected from the group consisting of hydroxypropylmethylcellulose, polyvinylpyrrolidone, and hydroxypropylcellulose, and a pH modifier for the manufacture of a composition providing a measurable improvement in dissolution rate of the 1-(6-amino-3,5-difluoropyridin-2-yl)-8-chloro-6-fluoro-7-(3-hydroxyazetidin-1-yl)-4-oxo-1,4-dihydxoquinoline-3-carboxylic acid or a salt thereof for treating disease in a patient by administering thereto said composition.

3. A composition or use according to claim 1 or 2 wherein the salt of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid comprises meglumine.

4. A composition or use according to any one of claims 1-3 wherein the pH modifier is selected from the group consisting of NaOH, LiOH, KOH, Na₂CO₃, NaHCO₃, K₂CO₃, KHCO₃, NaH₂PO₄, Na₂HPO₄, Na₃PO₄. KH₂PO₄, K₂HPO₄, K₃PO₄, Ca(OH)₂, Mg(OH)₂, Zn(OH)₂, Al(OH)₃, pyridoxine, triethanolamine, ammonium hydroxide, cytosine, diethylamine, meglumine, ornithine, glycine, lysine; arginine, valine, proline, aspartic acid, alanine, asparagine, isoleucine, leucine, methionine, threonine, choline hydroxide, procaine, diethylethanolamine, glucosamine, guanine, nicotinamide, piperazine, guanidine, olamine, piperidine, triethylamine, tromethamine, benzathine, adenine, and mixtures thereof.

5. A composition or use according to any one of claims 1-4 providing at least a 5% increase in dissolution rate.

6. A composition or use according to any one of claims 1-5 wherein the dose of the drug is from 0.03 to 200 mg/kg body weight.

## Patentansprüche

1. Eine Zusammensetzung umfassend 1-(6-Amino-3,5-difluorpyridin-2-yl)-8-chlor-6-fluor-7-(3-hydroxyazetidin-1-yl)-4-oxo-1,4-dihydrochinolin-3-carbonsäure oder ein Salz davon, einen Kristallisationshemmer gewählt aus der Gruppe bestehend aus Hydroxypropylmethylzellulose, Polyvinylpyrrolidon und Hydroxypropylzellulose, und einen pH-Modifikator, wobei die Zusammensetzung eine messbare Verbesserung in der Auflösungsgeschwindigkeit der 1-(6-Amino-3,5-difluorpyridin-2-yl)-8-chlor-6-fluor-7-(3-hydroxyazetidin-1-yl)-4-oxo-1,4-dihydrochinolin-3-carbonsäure oder des Salzes davon bereitstellt.

2. Verwendung von 1-(6-Amino-3,5-difluorpyridin-2-yl)-8-chlor-6-fluor-7-(3-hydroxyazetidin-1-yl)-4-oxo-1,4-dihydrochinolin-3-carbonsäure oder eines Salzes davon, eines Kristallisationshemmers gewählt aus der Gruppe bestehend aus Hydroxypropylmethylzellulose, Polyvinylpyrrolidon und Hydroxypropylzellulose, und eines pH-Modifikators für die Herstellung einer Zusammensetzung, die eine messbare Verbesserung in der Auflösungsgesch der 1-(6-Amino-3,5-difluorpyridin-2-yl)-8-chlor-6-fluor-7-(3-hydroxyazetidin-1-yl)-4-oxo-1,4-dihydrochinolin-3-carbonsäure oder eines Salzes davon bereitstellt, zur Behandlung einer Krankheit in einem Patienten durch Verabreichung der Zusammensetzung an diesen.

3. Eine Zusammensetzung oder eine Verwendung gemäß Ansprüchen 1 oder 2, worin das Salz der 1-(6-Amino-3,5-difluorpyridin-2-yl)-8-chlor-6-fluor-7-(3-hydroxyazetidin-1-yl)-4-oxo-1,4-dihydrochinolin-3-carbonsäure Meglumin umfasst.

4. Eine Zusammensetzung oder eine Verwendung gemäß irgendeinem der Ansprüche 1 bis 3, worin der pH-Modifikator gewählt ist aus der Gruppe bestehend aus NaOH, LiOH, KOH, Na₂CO₃, NaHCO₃, K₂CO₃, KHCO₃, NaH₂PO₄, Na₂HPO₄, Na₂PO₄, KH₂PO₄, K₂HPO₄, K₃PO₉, Ca(OH)₂, Mg(OH)₂, Zn(OH)₂, Al(OH)₃, Pyridoxin, Triethanolamin, Ammoniumhydroxid, Cytosin, Diethylamin, Meglumin, Ornithin, Glycerin, Lysin, Arginin, Valin, Prolin, Aspartinsäure, Alanin, Asparagin, Isoleucin, Leucin, Methionin, Threonin, Cholinhydroxid, Procain, Diethylethanolamin, Glucosamin, Guanin, Nicotinamid, Piperazin, Guanidin, Olamin, Piperidin, Triethylamin, Tromethamin, Benzathin, Adenin und Mischungen daraus.

5. Eine Zusammensetzung oder eine Verwendung gemäß irgendeinem der Ansprüche 1 - 4, die einen mindestens 5%igen Anstieg in der Auflösungsgeschwindigkeit bereitstellt.

6. Eine Zusammensetzung oder eine Verwendung gemäß irgendeinem der Ansprüche 1 - 5, wobei die Dosis des Arzneistoffs von 0,03 bis 200 mg/kg Körpergewicht ist.

## Revendications

1. Composition comprenant de l'acide 1-(6-amino-3,5-difluoro-pyridin-2-yl)-8-chloro-6-fluoro-7-(3-hydroxyazétidin-1-yl)-4-oxo-1,4-dihydroquinoléine-3-carboxylique ou un sel de celui-ci, un inhibiteur de cristallisation choisi dans le groupe consistant en l'hydroxypropylméthylcellulose, la polyvinylpyrrolidone et l'hydroxypropylcellulose, et un modificateur de pH, ladite composition offrant une amélioration mesurable dans la vitesse de dissolution de l'acide 1-(6-amino-3,5-difluoropyridin-2-yl)-8-chloro-6-fluoro-7-(3-hydroxyazétidin-1-yl)-4-oxo-1,4-dihydroquino-léine-3-carboxylique ou d'un sel de celui-ci.

2. Utilisation de l'acide 1-(6-amino-3,5-difluoropyridin-2-yl)-8-chloro-6-fluoro-7-(3-hydroxyazétidin-1-yl)-4-oxo-1,4-dihydroquinoléine-3-carboxylique ou d'un sel de celui-ci, d'un inhibiteur de cristallisation choisi dans le groupe consistant en l'hydroxypropylméthylcellulose, la polyvinylpyrrolidone et l'hydroxypropylcellulose, et d'un modificateur de pH pour la fabrication d'une composition offrant une amélioration mesurable dans la vitesse de dissolution de l'acide 1-(6-amino-3,5-difluoropyridin-2-yl)-8-chloro-6-fluoro-7-(3-hydroxyazétidin-1-yl)-4-oxo-1,4-dihydroquinoléine-3-carboxylique ou d'un sel de celui-ci pour traiter une maladie chez un patient par administration à celui-ci de ladite composition.

3. Composition ou utilisation selon la revendication 1 ou 2 où le sel de l'acide 1-(6-amino-3,5-difluoropyridin-2-yl)-8-chloro-6-fluoro-7-(3-hydroxyazétidin-1-yl)-4-oxo-1,4-dihydroquinoléine-3-carboxylique comprend la méglumine.

4. Composition ou utilisation selon l'une quelconque des revendications 1-3 où le modificateur de pH est choisi dans le groupe consistant en NaOH, LiOH, KOH, Na₂CO₃, NaHCO₃, K₂CO₃, KHCO₃, NaH₂PO₄, Na₂HPO₄, Na₃PO₄, KH₂PO₄, K₂HPO₄, K₃PO₄, Ca(OH)₂, Mg(OH)₂, Zn(OH)₂, Al(OH)₃, pyridoxine, triéthanolamine, hydroxyde d'ammonium, cytosine, diéthylamine, méglumine, ornithine, glycine, lysine, arginine, valine, proline, acide aspartique, alanine, asparagine, isoleucine, leucine, méthionine, thréonine, hydroxyde de choline, procaïne, diéthyléthanolamine, glucosamine, guanine, nicotinamide, pipérazine, guanidine, olamine, pipéridine, triéthylamine, trométhamine, benzathine, adénine, et leurs mélanges.

5. Composition ou utilisation selon l'une quelconque des revendications 1-4 offrant au moins une augmentation de 5 % de la vitesse de dissolution.

6. Composition ou utilisation selon l'une quelconque des revendications 1-5 où la dose du médicament est de 0,03 à 200 mg/kg de poids corporel.
